# EUROPEAN PATENT APPLICATION

(11) **EP 3 502 230 A1**
(43) Date of publication of application: **26.06.2019**
(21) Application number: 17210399.6
(22) Date of filing: 22.12.2017
(51) Int. Cl.: C12M 1/107, C02F 3/28, C12M 1/34, C02F 11/04, C12P 5/02

(54) **METHOD FOR CONTROLLING THE DOSAGE OF A BIOGAS PRODUCTION OPTIMIZER IN AN ANAEROBIC DIGESTER SLUDGE AND ANAEROBIC BIOGAS DIGESTER SYSTEM FOR PERFORMING SUCH A METHOD**

(71) Applicant: Yara International ASA, 0277 Oslo (NO)
(72) Inventor: DOPPELBAUER, Günter, 47445 Moers (DE)
(74) Representative: De Clercq & Partners

(57) **Abstract**

The present application relates to a method for controlling the dosage of a biogas production optimizer (BPO) in an anaerobic digester sludge from which biogas comprising hydrogen and methane is produced. The method comprises the steps of dosing the BPO to the anaerobic digester sludge and monitoring the concentration of the hydrogen in the produced biogas. The method further comprises the step of adjusting the dosage of the BPO according to the monitored concentration of the hydrogen in the produced biogas. The present application further relates to an anaerobic biogas digester system arranged to perform the method according to the present application.

## Description

### Field of the application

This application relates to the field of biogas production in anaerobic digester systems, more in particular to the control of the dosage of an optimizer for biogas production comprising hydrogen and methane.

### Background

Biogas, which is mainly composed of between 50 and 65 volume% of methane and between 35 and 50 volume% of carbon dioxide, is because of the favorable combustion properties of methane, a commercially valuable fuel, and can be obtained via microbial fermentation (enzymatic) processes. In addition, proper biogas production is required for stabilization of municipal, industrial and agricultural wastes via anaerobic digestion. It manages waste and/or releases energy. Many euryarchaeotal microorganisms can use hydrogen and carbon dioxide to produce biogas.

Anaerobic digester systems are among others used to treat wastes, energy crops, sludge, etc. and produce renewable energy. In this process, selected microorganisms are contacted with the waste which converts it into biogas.

Anaerobic digestion is a series of processes in which microorganisms break down biodegradable material in the absence of oxygen. The digestion process begins with bacterial hydrolysis of input biomass in order to break down insoluble organic polymers such as carbohydrates and proteins into sugars and amino acids. Acidogenic bacteria then convert the sugars and amino acids into carbon dioxide, hydrogen, ammonia and organic acids. Acetogenic bacteria then convert these resulting organic acids into acetic acid and produce additional ammonia, hydrogen and carbon dioxide. Finally, methanogens convert those products into methane and carbon dioxide. Anaerobic digestion is a multistep process in which different microbial communities are working in syntrophy. If the syntrophy is disrupted by organic overload or other changes such as temperature, pH or toxins, then the entire process may slow down or stop, causing costly delays at agricultural, industrial, and municipal treatment plants. Most notably, efficient metabolism of hydrogen (H₂) and propionic acid is required. Propionate and H₂-accumulation have been seen as an indicator of process imbalance (Anaerobic waste water treatment, McCarty and Smith, Environ. Sci. Technol., 1986, 20 (12), pp 1200-1206).

One way of increasing methane production in anaerobic digester systems is "bioaugmentation", in which specific, active microbes are added to the anaerobic digester system to enhance process performance. In WO 2011/123854 for instance, a bioaugmentation system is described adding hydrogenotrophic methanogens to the anaerobic digester system to enhance methane production.

WO 2011/112736 relates to a process and apparatus for producing biogas having a high methane content. The focus of the application is on providing optimal values for the oxidation-reduction potential, the pH and the temperature of the reactor bulk liquid for an anaerobic process producing biogas, and also monitoring those values and adjusting the process if necessary.

A Biogas Production Optimizer (BPO) is used to optimize the efficiency of fermentation in biogas plants by shifting the oxidation-reduction potential in micro-scale from anaerobic towards anoxic conditions. Anaerobic conditions are conditions where there is total absence of free oxygen (O₂) or bound oxygen (NO₂⁻, NO₃⁻), while anoxic conditions are conditions where there is absence of free oxygen but presence of bound oxygen. This part-time anoxic metabolism provides slightly higher energy yield to fermentative bacteria than a constant anaerobic metabolism. The fermenting bacteria are given higher accessibility to the carbon sources in the substrate, thus accelerating and optimizing the conversion of substrates into biogas, resulting in a higher energy content. An example of such a BPO is a nitrate solution.

Today, the BPO dosage level is normally estimated via the biogas production or alternatively via the organic content of the feed material and is just a prediction. Both pathways indicate a dosage level that needs to be proven to be optimal. The only control mechanism so far is to limit the temperature increase in the anaerobic digester (also called a fermenter), but this is a slow process, and thus is only useful for emergency shut down procedures.

There are currently no prior art documents that disclose an easy and reliable control parameter for dosage of BPO in an anaerobic digester system. Therefore, the goal of this application is to provide a method for controlling the BPO dosage in an anaerobic digester sludge from which biogas comprising hydrogen and methane is produced in order to optimize methane production in the produced biogas, in which an easy and reliable control parameter can be used to determine and control the BPO dosage.

### Summary of the application

According to an aspect of the present application, a method is disclosed for controlling the dosage of a BPO in an anaerobic digester sludge from which biogas comprising hydrogen and methane is produced, the method comprises the steps of dosing the BPO to the anaerobic digester sludge and monitoring the concentration of the hydrogen in the produced biogas, wherein the method further comprises the step of adjusting the dosage of BPO in accordance with the monitored concentration of the hydrogen.

The advantage of using the hydrogen concentration in the produced biogas to enable control of the BPO dosage is that it is an easy measurable parameter. It is furthermore found that by monitoring the hydrogen concentration in the produced biogas and adjusting the concentration of the BPO according to this monitored concentration of hydrogen, optimal and stable methane production from the anaerobic digester sludge is obtained, depending on the real conditions in the fermenter and independently from the various feed materials and microbial communities used, resulting in a reliable way to optimize methane production, instead of using a prediction as optionally done in the prior art.

In a possible method according to the application, the dosage of BPO is adjusted when the hydrogen concentration in the biogas is above 100 ppm, in particular above 200 ppm and more in particular above 500 ppm. Hydrogen is a very reactive component that can damage piping. Furthermore, the hydrogen has negative influence on the digestion by the microorganisms. Therefore, the lower the H₂ concentration, the better.

In an optional method according to the application, the dosage of BPO is adjusted in such a way that if the monitored concentration of the hydrogen in the produced biogas rises by 10 to 30% without dosage of the BPO, the dosage of the BPO to the anaerobic digester sludge is decreased stepwise by 10% until the hydrogen concentration is at an acceptable level.

In a possible method according to the application, an aqueous sodium, potassium, calcium, ammonium or magnesium nitrate solution or a nitric acid solution is dosed as the BPO to the anaerobic sludge and the dosage thereof is adjusted in accordance with the monitored concentration of the hydrogen in the produced biogas.

In a possible method according to the application, the aqueous nitrate solution or nitric acid solution is dosed to the anaerobic sludge in an amount between 2 and 6 mg nitrate per m³ biogas and the dosage thereof is adjusted in accordance with the monitored concentration of the hydrogen in the produced biogas.

In a possible method according to the present application, the concentration of the hydrogen in the produced biogas is monitored approximately every hour, and more in particular approximately every two hours. This is a sufficient frequency to follow up the hydrogen concentration.

In a potential method according to the application, the anaerobic sludge treatment process is selected from a municipal sludge treatment plant, an agricultural biogas plant, an industrial anaerobic sludge digestion plant or any other anaerobic process plant.

According to a further aspect of the present application, an anaerobic biogas digester system is disclosed, comprising an anaerobic digester reactor comprising an anaerobic digester sludge and a biogas on top of the anaerobic digester sludge, comprising methane and hydrogen, means for monitoring the hydrogen concentration in the biogas and a BPO dosage unit configured to dose the BPO, wherein the anaerobic biogas digester further comprises a controller that controls the BPO dosage unit for adjusting the dosage of BPO in accordance with the monitored concentration of the hydrogen in the produced biogas.

In a possible embodiment of a system according to the application, the anaerobic biogas digester system is capable of performing a method according to the present application as described above.

### Brief description of the figures

In FIG. 1, the variations of the hydrogen concentration in the biogas produced from a digester sludge and of the dosage of the Biogas Production Optimizer (BPO) to the digester sludge over a period of 9 months are shown;

In FIG. 2, the variations of the hydrogen concentration in the biogas produced from a digester sludge and of the dosage of the biogas production optimizer (BPO) to the digester sludge over a period of 2 months are shown.

### Detailed description

As defined herein, the singular forms "a", "an", "the" include both the singular and the plural, unless the context clearly indicates otherwise. The terms "comprise", "comprises" as used below are synonymous with "including", "include" or "contain", "contains" and are inclusive or open and do not exclude additional unmentioned parts, elements or method steps. Where this description refers to a product or process which "comprises" specific features, parts or steps, this refers to the possibility that other features, parts or steps may also be present, but may also refer to embodiments which only contain the listed features, parts or steps. All references cited in this description are hereby deemed to be incorporated in their entirety by way of reference.

In a method according to the present application, the dosage of a Biogas Production Optimizer (BPO) to an anaerobic digester sludge is adjusted according to the monitored concentration of hydrogen in the biogas produced from the digester sludge. This method comprises the consequent steps of first dosing BPO to the anaerobic digester sludge, subsequently, measuring the concentration of the hydrogen in the produced biogas, and finally, adjusting the BPO according to the monitored concentration of the hydrogen in the produced biogas.

As defined herein, a BPO is a solution used to optimize the efficiency of fermentation in biogas plants by shifting the oxidation-reduction potential in micro-scale digestion from anaerobic towards anoxic conditions. This shift towards anoxic conditions metabolism provides slightly higher energy yield to fermentative bacteria than a constant anaerobic metabolism. The fermenting bacteria are given higher accessibility to the carbon sources in the substrate, thus accelerating and optimizing the conversion of substrates into biogas, resulting in a higher energy content.

A nitrate solution is very suitable to be used as BPO. The nitrate present in the nitrate solution is added as an electron acceptor to facilitate methane production in anaerobic sludge and waste water treatment processes, thus having a similar effect to the addition of oxygen as an electron acceptor. The dosage of a nitrate solution will support the microbial activity in general. There are a number of microorganisms that are involved in the process of anaerobic digestion including acetic acid forming bacteria (acetogens) and methane forming bacteria (methanogens), resulting in that organic waste is converted to intermediate molecules including sugars, hydrogen, organic acids such as acetic acid and propionic acid, and finally to biogas. The nitrate added to the initial degradation steps leads to more biomass in forms of anoxic species that later on can be degraded anaerobically. Additionally, the organic matter itself will be more easily degradable due to enhancement of these initial processes.

In particular, the BPO is selected from an aqueous solution of sodium nitrate, magnesium nitrate, potassium nitrate, ammonium nitrate, calcium nitrate or a nitric acid solution. More in particular, the BPO is an aqueous solution of calcium nitrate. Optionally, the BPO can be an aqueous sodium nitrate, magnesium nitrate, ammonium nitrate or calcium nitrate solution produced by adding sodium nitrate, magnesium nitrate, potassium nitrate, ammonium nitrate or calcium nitrate to water in such a way that the resulting concentration of nitrate is such that the BPO can be added to the digester sludge in an amount of 2 to 6 mg per m³ of biogas. Optionally, the BPO can be an nitric solution in which the nitrate concentration is such the BPO can be added to the digester sludge in an amount of 2 to 6 mg per m³ of biogas.

As defined herein, with an anaerobic digester sludge is meant a sludge or waste water from municipal sewage disposal plants, manure treatment processes, organics from industrial operations (such as chemical and biochemical processing), food industry, paper and pulp operations. Any sludge or waste water being useful as a source for methane production in an anaerobic process may be used in the anaerobic digestion process according to the present application. In particular, "an anaerobic sludge" means sludge from a municipal sludge treatment plant, from an agricultural biogas plant, from an industrial anaerobic sludge digestion plant or from any other anaerobic process plant.

Anaerobic digester sludge systems and waste water treatment systems can be constructed differently in respect of involving a one-stage or single stage process, a two-stage or a multi-stage process. In this respect, a single step process is a process wherein all the biological reactions happen within one single sealed anaerobic digester. As used herein, an anaerobic digester is a reactor comprising the biogas digester sludge and the biogas produced from the digester sludge and present above the digester sludge. A two-stage or multi stage process involves the use of more than one anaerobic digester, and wherein each reactor is respect to providing optimal conditions for the biological processes to be conducted within each anaerobic digester. For example, the first digestion processes, the hydrolysis and the acidogenesis steps may occur in one anaerobic digester, whereas the next steps, the acetogenesis and the methanogenesis, occur in another anaerobic digester. The process according to the present application is applicable in both single and multi-stage processes for the biological production of methane.

Anaerobic digester sludge systems and waste water treatment systems may also be designed and engineered under the form of a number of different process configurations, e.g. as discontinuous batch systems or continuous systems. A batch system is the simplest form, wherein the substrate to be digested is added to a reactor in one batch and then is sealed for the duration of the process. In continuous digestion systems, the substrate to be digested is continuously added to the reactor, and the end products, i.e. biogas and other degradation products are continuously removed, resulting in continuous and relatively constant production of biogas. Both single and multiple digester reactors placed in sequence are commonly used. Examples of well-known systems for continuous treatment of sludge and waste water include continuous stirred-tank reactors (CSTRs), up flow anaerobic sludge blankets (UASB), Expanded granular sludge beds (EGSB) and Internal circulation reactors (IC). For anaerobic digesters working with a continuous flow, a periodical or constant dosage of inorganic nitrate is necessary. Control of continuously working processes may demand a control system with process control parameters enabling adjustment of the BPO dosage. The present application is applicable to all the various continuous anaerobic sludge and waste water treatment processes as discussed above.

It has been found that optimal and stable methane production can be achieved by monitoring the concentration of hydrogen in the biogas produced from the digester sludge and by adjusting the BPO dosage to the sludge according to those measurements of the hydrogen concentration in the produced biogas. Thus, according to the present application, monitoring of the concentration of hydrogen in the produced biogas from the digester sludge is performed during continuous flow working sludge and waste water treatment and the dosage of BPO to the sludge is adjusted according to those measurements.

It has been found that, in particular, the monitored concentration of hydrogen in the produced biogas should be kept below 500 ppm, more in particular below 200 ppm and most in particular below 100 ppm.

The concentration of hydrogen in the produced biogas can be monitored approximately every hour or every two hours. As an example, a gas analyzer carries out once an hour or every two hours a whole sequence of measurements of components in the produced biogas such as CH₄, CO₂, O₂, H₂S and H₂, which takes several minutes. Then the gas analyzer pauses and starts again with the measuring cycle. Doing so, some 10 - 20 measurements per day will be achieved and this is a sufficient dense frequency to follow up the hydrogen concentration.

An anaerobic digester system according to the present application comprises an anaerobic digester sludge and a biogas on top of the anaerobic digester sludge, the biogas comprising methane, hydrogen, CO₂ and trace gases. The system further comprises means for monitoring the hydrogen concentration. As defined herein, means for monitoring the hydrogen concentration can be a gas analyzer equipped with a hydrogen sensor for monitoring the concentration of hydrogen in the gases in contact with the hydrogen sensor. For example, AWITE gas analyzers equipped with hydrogen sensors are suitable means for measuring the concentration of hydrogen in the biogas produced from the digester sludge according to the present application.

The anaerobic digester system further comprises a dosage unit configured to dose the BPO to the digester sludge. In addition, the anaerobic gas digester is equipped with a controller that controls the BPO dosage unit for adjusting the BPO dosage to the digester sludge according to the monitored concentration of hydrogen in the biogas produced from the digester sludge. The anaerobic digester system is capable of performing the method disclosed in this application.

### Example:

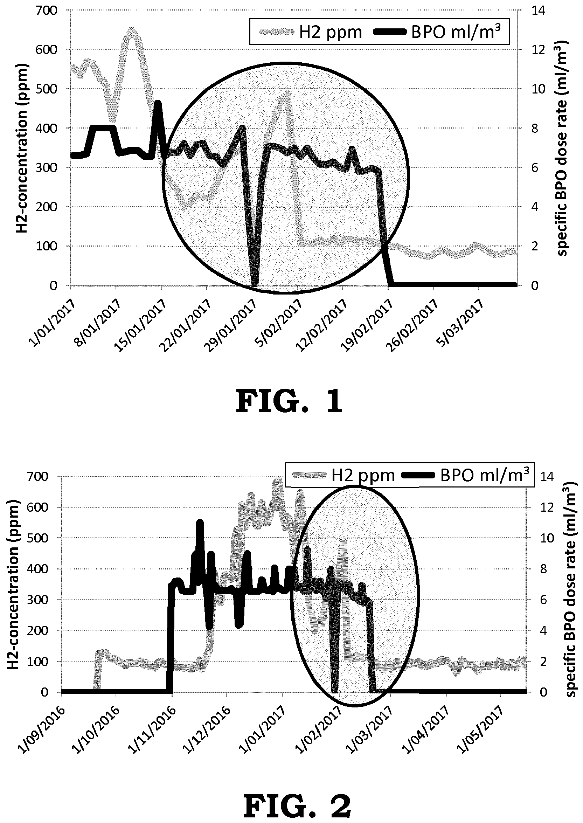

A test was conducted on a full scale at a customer site producing methane and hydrogen from an anaerobic digester sludge, wherein the hydrogen concentration in the biogas produced from the digester sludge was monitored over time using a hydrogen sensor that is integrated in a gas-analyzer. A biogas production optimizer (BPO) solution was prepared by dissolving calcium nitrate salts in water in such a way that 45 % of the weight of the resulting solution accounted for the dissolved calcium nitrate salts resulting in a 45 w/w% aqueous calcium nitrate solution. The BPO solution was dosed to the digestion sludge. Variations of the hydrogen concentration in the biogas produced from the digester sludge and of the dosage of the BPO to the digester sludge are shown in Figure 1, over a period of 9 months. The variations of the hydrogen concentration in the biogas produced from the digester sludge and of the dosage of BPO to the digester sludge over a more specific period of 2 months, during which the BPO dosage was gradually reduced to return to the preferred concentration of hydrogen of 100 ppm, is shown in Figure 2. Out of FIG. 2, it can be concluded that when the dosage of BPO is gradually decreased between 1 February 2017 and 17 February 2017, from approximately 7 ml of BPO/ m³ biogas produced to 6 ml of BPO/ m³ biogas produced, a stable, optimal hydrogen concentration of 100 ppm is achieved.

## Claims

1. Method for controlling the dosage of a biogas production optimizer (BPO) in an anaerobic digester sludge from which biogas comprising hydrogen and methane is produced, the method comprising the steps of:
• dosing the BPO to the anaerobic digester sludge, and
• monitoring the concentration of the hydrogen in the produced biogas
wherein the method further comprises the step of adjusting the dosage of BPO according to the monitored concentration of the hydrogen in the produced biogas.

2. Method according to claim 1, wherein the method comprises the step of adjusting the dosage of BPO when the hydrogen concentration in the produced biogas is above 100 ppm, in particular above 200 ppm and more in particular above 500 ppm.

3. Method according to claim 2, wherein the method comprises the step of adjusting the dosage of BPO in such a way that, if the monitored concentration of the hydrogen in the produced biogas rises by 10 to 30% without dosage of the BPO, the dosage of the BPO to the anaerobic digester sludge is decreased stepwise by 10% until the hydrogen concentration is at an acceptable level.

4. Method according to any one of claims 1 to 3, wherein the method comprises the steps of
• dosing an aqueous sodium, potassium, calcium, ammonium or magnesium nitrate solution or a nitric acid solution as the BPO to the anaerobic sludge, and
• adjusting the dosage thereof in accordance with the monitored concentration of the hydrogen in the produced biogas.

5. Method according to claim 4, wherein the method comprises the steps of
• Adding the aqueous nitrate solution or the nitric acid solution the anaerobic sludge in an amount between 2 and 6 mg nitrate per m³ biogas, and
• adjusting the dosage thereof in accordance with the monitored concentration of the hydrogen in the produced biogas.

6. Method according to any one claims 1 to 5, wherein the method comprises the step of monitoring the concentration of the hydrogen in the produced biogas approximately every hour.

7. Method according to any one of claims 1 to 6, wherein the method comprises the step of monitoring the concentration of the hydrogen in the produced biogas approximately every two hours.

8. Method according to any one of claims 1 to 7, wherein the anaerobic sludge treatment process is selected from a municipal sludge treatment plant, an agricultural biogas plant, an industrial anaerobic sludge digestion plant or any other anaerobic process plant.

9. An anaerobic biogas digester system comprising :
• an anaerobic digester reactor comprising an anaerobic digester sludge and a biogas on top of the anaerobic digester sludge, the biogas comprising methane and hydrogen,
• means for monitoring the hydrogen concentration in the produced biogas,
• a biogas production optimizer (BPO) dosage unit configured to dose the BPO to the anaerobic digester sludge,
wherein the anaerobic biogas digester further comprises a controller that controls the BPO dosage unit for adjusting the dosage of BPO in accordance with the monitored concentration of the hydrogen in the produced biogas.

10. An anaerobic biogas digester system according to claim 9 for performing a method according to any one of claims 1 to 8.
